# EUROPÄISCHE PATENTSCHRIFT

(11) **EP 2 942 008 B1**
(45) Veröffentlichungstag und Bekanntmachung des Hinweises auf die Patenterteilung: **27.02.2019**
(21) Anmeldenummer: 15163118.1
(22) Anmeldetag: 10.04.2015
(51) Int. Cl.: A61B 5/08, A61B 5/0205, A61B 5/00, A61B 5/05, A61B 5/11, G01V 3/175, G01S 13/52, G01S 13/88, G01R 33/36, G01R 33/28, A61B 5/055, G01R 33/34, G01R 33/567, G01R 33/20

(54) **MAGNETRESONANZVORRICHTUNG MIT EINER BEWEGUNGSERFASSUNGSEINHEIT SOWIE EIN VERFAHREN ZU EINER ERFASSUNG EINER BEWEGUNG EINES PATIENTEN WÄHREND EINER MAGNETRESONANZUNTERSUCHUNG**
MAGNETIC RESONANCE DEVICE WITH A MOVEMENT DETECTING UNIT AND A METHOD FOR RECORDING A MOVEMENT OF A PATIENT DURING A MAGNETIC RESONANCE INVESTIGATION
DISPOSITIF DE RÉSONANCE MAGNÉTIQUE DOTÉ D'UNE UNITÉ DE DÉTECTION DE MOUVEMENT ET PROCÉDÉ DE DÉTECTION D'UN MOUVEMENT D'UN PATIENT PENDANT UN EXAMEN PAR RÉSONANCE MAGNÉTIQUE

(30) Priorität: 07.05.2014 DE 102014208537
(43) Veröffentlichungstag der Anmeldung: 11.11.2015
(73) Patentinhaber: Siemens Healthcare GmbH, 91052 Erlangen (DE)
(72) Erfinder: Biber, Stephan, 91056 Erlangen (DE); Fackelmeier, Andreas, 91177 Thalmässing (DE); Huber, Klaus, 91090 Effeltrich (DE); Rehner, Robert, 91077 Neunkirchen am Brand (DE)

(56) Entgegenhaltungen:
- DE-A1-102008 006 711
- DE-A1-102009 021 232
- US-A1- 2010 106 008
- US-A1- 2010 152 600
- XU YONG ET AL: "An Overview of Ultra-Wideband Technique Application for Medial Engineering", COMPLEX MEDICAL ENGINEERING, 2007. CME 2007. IEEE/ICME INTERNATIONAL C ONFERENCE ON, IEEE, PI, 1. Mai 2007 (2007-05-01), Seiten 408-411, XP031159958, ISBN: 978-1-4244-1077-4

## Beschreibung

Die Erfindung geht aus von einer Magnetresonanzvorrichtung mit einer Hochfrequenzeinheit, die eine Hochfrequenzantenne, zumindest eine Hochfrequenzleitung und zumindest eine Hochfrequenzeinkoppelstelle aufweist, wobei mittels der zumindest einen Hochfrequenzleitung Hochfrequenzsignale an die Hochfrequenzantenne übertragen werden und an der zumindest einen Hochfrequenzeinkoppelstelle in die Hochfrequenzantenne eingespeist werden, einem Patientenaufnahmebereich, der zumindest teilweise von der Hochfrequenzantenne umgeben ist, und einer Bewegungserfassungseinheit zu einer Erfassung einer Bewegung eines innerhalb des Patientenaufnahmebereichs positionierbaren Patienten.

Eine Magnetresonanzbildgebung umfasst vorzugsweise mehrere Sende-Empfangszyklen, die durch eine Nachverarbeitung zu einem Bild zusammengesetzt werden. Bei sich bewegenden Körperbereichen eines Patienten, beispielsweise aufgrund eines Herzschlags und/oder einer Atmung des Patienten, muss die Bilderfassung stets in der gleichen Phase der Bewegung erfolgen. Hierzu werden für die Magnetresonanzbildgebung Triggersignale aus der Köperbewegung abgeleitet, die einen Triggerzeitpunkt für die Bilderfassung angeben bzw. es werden auch Bilddaten, die während einer unerwünschten Bewegung des Patienten erfasst wurden, verworfen.

Bisher werden zur Erfassung einer Bewegung des Patienten externe Messvorrichtungen verwendet. Beispielsweise wird zu einer Erfassung einer Atembewegung des Patienten während einer Magnetresonanzbildgebung ein Atemkissen verwendet, dass die Atembewegung anhand einer Luftdruckänderung erfasst. Des Weiteren wird beispielsweise zu einer Erfassung einer Herzbewegung des Patienten während einer Magnetresonanzbildgebung Elektroden verwendet. Diese externen Messvorrichtungen sind jedoch sehr Kosten intensiv und verursachen zudem einen hohen Vorbereitungsaufwand für ein die Magnetresonanzbildgebung betreuendes Bedienpersonal, wie beispielsweise eine entsprechende Anbringung der externen Messvorrichtung an dem Patienten.

DE 10 2008 006 711 A1 beschreibt eine medizinische Diagnose- oder Therapieeinheit mit mindestens einem 3-D-Radararray zur Detektion von Orts- und/oder Bewegungsdaten van Objekten in einem Untersuchungsraum der Diagnose- oder Therapieeinheit.

DE 10 2009 021 232 A1 beschreibt eine Patientenliege für ein bildgebendes medizinisches Gerat mit einer Patientenlagerungsplatte, welche wenigstens eine Radar-Antenne zur Gewinnung physiologischer und/oder geometrischer Daten eines auf der Patientenlagerungsplatte gelagerten Patienten aufweist.

US 2010/0106008 A1 beschreibt ein Magnetresonanzgerät mit einer ersten HF-Spule zur Aufnahme von Magnetresonanzdaten eines Untersuchungsobjektes und mehreren Messelementen zur Aufnahme von Daten über eine Bewegung des Untersuchungsobjektes. Der vorliegenden Erfindung liegt insbesondere die Aufgabe zugrunde, eine besonders einfache und Kosten sparende Erfassung einer Patientenbewegung während einer Magnetresonanzuntersuchung bereitzustellen. Die Aufgabe wird durch die Merkmale der unabhängigen Ansprüche gelöst. Vorteilhafte Ausgestaltungen sind in den Unteransprüchen beschrieben.

Die Erfindung geht aus von einer Magnetresonanzvorrichtung mit einer Hochfrequenzeinheit, die eine Hochfrequenzantenne, zumindest eine Hochfrequenzleitung und zumindest eine Hochfrequenzeinkoppelstelle aufweist, wobei mittels der zumindest einen Hochfrequenzleitung Hochfrequenzsignale an die Hochfrequenzantenne übertragen werden und an der zumindest einen Hochfrequenzeinkoppelstelle in die Hochfrequenzantenne eingespeist werden, einem Patientenaufnahmebereich, der zumindest teilweise von der Hochfrequenzantenne umgeben ist, und einer Bewegungserfassungseinheit zu einer Erfassung einer Bewegung eines innerhalb des Patientenaufnahmebereichs positionierbaren Patienten. Die Hochfrequenzsignale werden über die Hochfrequenzantenne als hochfrequente Magnetresonanzsequenzen in den Patientenaufnahmebereich eingestrahlt.

Die zumindest eine Hochfrequenzleitung weist zumindest ein Einkopplungselement auf, mittels dessen zumindest ein Bewegungserfassungssignal der Bewegungserfassungseinheit in die Hochfrequenzleitung einkoppelt. Es kann derart besonders einfach das Signal zur Erfassung der Bewegung des Patienten an die Hochfrequenzantenne übertragen und von dort ausgesendet werden. Weiterhin kann hierdurch die Bewegungserfassungseinheit besonders kompakt und Platz sparend innerhalb der Magnetresonanzvorrichtung, insbesondere innerhalb der Hochfrequenzantenneneinheit integriert werden, da auf eine separate Sendeantenne der Bewegungserfassungseinheit verzichtet werden kann. Die erfindungsgemäße Ausgestaltung ermöglicht es zudem, dass eine Vorbereitung des Patienten für eine Magnetresonanzuntersuchung besonders Zeit sparend erfolgt, da auf die Verwendung von externen Einheiten zur Erfassung der Bewegung des Patienten und deren Anordnung am Patienten verzichtet werden kann. Zudem entfällt durch diese Ausgestaltung des Magnetresonanzgeräts eine Zeit aufwendige Reinigung der externen Einheiten zur Erfassung der Bewegung des Patienten. Des Weiteren kann aufgrund der zumindest teilweisen Integration der Bewegungserfassungseinheit in die Hochfrequenzeinheit eine einfache und Kosten günstige Bewegungserfassung bei jeder Magnetresonanzuntersuchung erfolgen, insbesondere auch bei Magnetresonanzuntersuchungen, bei denen dies bisher nicht unbedingt erforderlich war und/oder aus Kostengründen nicht durchgeführt wurde.

Die Hochfrequenzeinheit kann zudem auch mehr als eine Hochfrequenzleitung und auch mehr als eine Hochfrequenzeinkoppelstelle aufweisen, wobei vorzugsweise eine Anzahl der Hochfrequenzleitungen einer Anzahl an Hochfrequenzeinkoppelstellen entspricht, so dass für jede Hochfrequenzleitung eine eigene Hochfrequeneinkoppelstelle zur Verfügung steht. Die Hochfrequenzleitungen umfassen bevorzugt Koaxialkabel. Grundsätzlich ist auch eine davon abweichende Ausbildung der Hochfrequenzleitungen denkbar.

Des Weiteren weist die Bewegungserfassungseinheit eine Radareinheit auf und eine Bewegung des Patienten wird mittels zumindest eines Radarsignals der Radareinheit erfasst. Die Verwendung der Radareinheit bietet den Vorteil, dass sie besonders Kosten günstig in die bereits bestehende Hochfrequenzeinheit integriert werden kann. Besonders vorteilhaft weist die Radareinheit eine Dopplerradareinheit auf, wobei eine Bewegung des Patienten mittels eines an dem Patienten reflektierten Radarsignals erfasst wird. Derart können auch geringfügige Bewegungen des Patienten, wie beispielsweie eine Atmung und/oder ein Herzschlag des Patienten, präzise erfasst werden. Zudem eignet sich die Dopplerradareinheit zur Erfassung eine über die Atmung und/oder den Herzschlag hinaus reichende Bewegung des Patienten zu erfassen, wie beispielsweise eine Bewegung eines Arms des Patienten.

Vorzugsweise werden mittels der Radareinheit, insbesondere der Dopplerradareinheit, Radarsignale generiert und mittels der Hochfrequenzeinheit, insbesondere der Hochfrequenzantenne, ausgesandt. Zumindest ein reflektiertes Radarsignal wird zudem von der Hochfrequenzeinheit, insbesondere der Hochfrequenzantenne, erfasst, wobei das reflektierte Radarsignal an einem, in dem Patientenaufnahmebereichs einbringbarem Untersuchungsobjekt, insbesondere an einem Patienten, reflektiert wird. Aufgrund des Aussendens und/oder Erfassens der Radarsignale mittels der Hochfrequenzantenne der Hochfrequenzeinheit kann vorteilhaft auf eine zusätzliche Antenne, insbesondere eine separate Radarantenne, verzichtet werden. Hierdurch können auch unerwünschte Wechselwirkungen zwischen einer separaten Radarantenne und der Hochfrequenzantenne vorteilhaft verhindert werden. Besonders vorteilhaft wird das erfasste Radarsignal von der Hochfrequenzantenne mittels der Hochfrequenzleitung und dem Einkopplungselement zu der Dopplerradareinheit geleitet und dort hinsichtlich einer Bewegung des Patienten anhand eines Dopplereffekts in den erfassten Signalen ausgewertet. Die Aussendung und/oder die Erfassung der Radarsignale mittels der Hochfrequenzantenne kann zudem zeitgleich zur Aussendung von Hochfrequenzsignalen erfolgen.

Eine unerwünschte Beeinflussung und/oder Störung zwischen dem Hochfrequenzsignal, das mit einer Frequenz von beispielsweise 123,2 MHz abgestrahlt wird, und dem Radarsignal kann vorteilhaft verhindert werden, wenn das Radarsignal eine Frequenz von mindestens 3 GHz umfasst. Besonders vorteilhaft weist das Radarsignal eine Frequenz von mindestens 4 GHz und besonders bevorzugt von mindestens 5 GHz auf. Vorzugsweise weist zudem das Radarsignal eine maximale Frequenz von 30 GHz, vorteilhaft von 25 GHz und besonders bevorzugt von 15 GHz auf. Besonders vorteilhaft ist das auszusendende Radarsignal schmalbandig ausgebildet, so dass das Radarsignal unempfindlich hinsichtlich parasitärer Effekte und/oder unempfindlich hinsichtlich einer niedrigen Hochfrequenzleistung des Hochfrequenzsignals und/oder unempfindlich hinsichtlich weiterer Störeinflüsse ist. Vorzugsweise weist das schmalbandige Radarsignal eine Breite von nur einigen Hz auf und bevorzugt eine Breite von kleiner 100 Hz.

Weiterhin wird vorgeschlagen, dass die Hochfrequenzeinheit zwei oder mehr Hochfrequenzleitungen und zwei oder Hochfrequenzeinkoppelstellen aufweist, wobei mittels der zwei oder mehr Hochfrequenzleitungen jeweils ein Hochfrequenzsignal zu einer der zwei oder mehr Hochfrequenzeinkoppelstellen übertragen wird und jede der zwei oder mehr Hochfrequenzleitungen ein Einkoppungselement zur Einspeisung eines Radarsignals in die Hochfrequenzantenne aufweist. Hierdurch kann ein für die Bewegung des Patienten relevanter Bereich aus unterschiedlichen Richtungen relativ zu vorzugsweie einem Körper des Patienten erfasst werden. Zudem kann eine Herzbewegung und/oder Atembewegung des Patienten hierdurch redundant erfasst werden. Zudem weist die Hochfrequenzantenne ein unbestimmtes Abstrahlverhalten an den unterschiedlichen Hochfrequenzeinkoppelstellen auf, so dass besonders vorteilhaft Störanteile der erfassten Radarsignale aus den Daten eliminiert werden können, da diese Störanteile aufgrund der redundanten Erfassung einfach bestimmbar sind. Des Weiteren können sich die zwei oder mehr Radarsignale, die mittels der zwei oder mehr Einkoppelelemente in die Hochfrequenzantenne eingespeist werden, hinsichtlich einer Radarfrequenz unterscheiden.

Weist die Bewegungserfassungseinheit eine Schalteinheit auf, wobei die Einspeisung der zwei oder mehr Radarsignale in die Hochfrequenzantenne mittels der Schalteinheit erfolgt, kann eine besonders einfache Einspeisung von Radarsignalen an unterschiedlichen Hochfrequenzeinkoppelstellen erreicht werden. Zudem kann hierdurch eine Kosten günstige Einspeisung der zwei oder mehr Radarsignale erfolgen. Die Schalteinheit kann beispielsweise eine Multiplexereinheit umfassen.

In einer weiteren Ausgestaltung der Erfindung wird vorgeschlagen, dass mittels einer einzigen Hochfrequenzeinkoppelstelle und einer einzigen Hochfrequenzleitung mit einem einzigen Einkoppelelement zwei oder mehr Radarsignale in die Hochfrequenzantenne eingespeist werden, wobei die zwei oder mehr Radarsignale hinsichtlich einer Radarfrequenz unterschiedlich ausgebildet sind. Mittels der unterschiedlichen Radarfrequenzen kann die Bewegung des Patienten, insbesondere eine Herzbewegung und/oder eine Atembewegung, redundant erfasst werden. Hierdurch können innerhalb der Übertragungsstrecke parasitäre Effekte, beispielsweise schlechten Transmissioneigenschaften der Hochfrequenzeinkoppelstelle für bestimmte Radarfrequenzen, während der Erfassung und/oder der Auswertung der erfassten Radarsignale eliminiert werden. Zudem weist die Hochfrequenzantenne ein unbestimmtes Abstrahlverhalten für die unterschiedlichen Radarfrequenzen auf, so dass besonders vorteilhaft Störanteile der erfassten Radarsignale aus den Daten eliminiert werden können, da diese Störanteile aufgrund der redundanten Erfassung einfach bestimmbar sind.

Weist die Bewegungserfassungseinheit eine Auswerteinheit auf, können zudem diese unterschiedlichen, erfassten Radarsignale miteinander verknüpft werden, beispielsweise ein Quadratmittelwert berechnet werden. Damit kann eine Verbesserung einer Erfassung einer Herzbewegung und/oder einer Atembewegung des Patienten erreicht werden. Hierbei kann ein großes Verhältnis von Nutzsignal zu Störsignal bereitgestellt werden, da die Störsignale näherungsweise als statistisch unabhängig betrachtet werden können und somit aus den erfassten Radarsignalen eliminiert werden können. Die erfassten Radarsignale können sich hinsichtlich einer Aussendefrequenz unterscheiden und/oder hinsichtlich eines Aussendeorts, beispielsweise aufgrund unterschiedlicher Hochfrequenzeinkoppelstellen, mittels derer die Radarsignale in die Hochfrequenzantenne eingespeist werden.

In einer weiteren Ausgestaltung der Erfindung wird vorgeschlagen, dass das Einkopplungselement einen Richtkoppler umfasst, wodurch ein besonders kompaktes Einkopplungselement zur Verfügung gestellt werden kann. In einer alternativen Ausgestaltung der Erfindung wird vorgeschlagen, dass das Einkopplungselement ein Hochpassfilterelement umfasst, wodurch eine vorteilhafte Signaleinkopplung des Radarsignals in die Hochfrequenzantenne erreicht werden kann. Vorzugsweise weist der Richtkoppler für Hochfrequenzsignale mit einer Frequenz von 123,2 MHz eine Dämpfung von 40 dB auf. Für Radarsignale, die eine Frequenz von einigen GHz aufweisen, weist der Richtkoppler dagegen eine deutlich niedrigere Dämpfung auf von beispielsweise 25 dB.

Weist die Bewegungserfassungseinheit zudem eine Anpassungseinheit auf, kann der Richtkoppler, insbesondere bei einer Einkopplung und/oder einer Übertragung eines zu übertragenden Radarsignals, vorteilhaft an eine Frequenz des zu übertragenden Radarsignals angepasst werden. Die Anpassungseinheit kann hierbei eine kapazitive Anpassungseinheit, die beispielsweise einen Kondensator umfasst, und/oder eine induktive Anpassungseinheit und/oder weitere, dem Fachmann als sinnvoll erscheinende Anpassungseinheiten umfassen.

Des Weiteren geht die Erfindung aus von einem Verfahren zu einer Erfassung einer Bewegung, insbesondere einer Herzbewegung und/oder einer Atembewegung, eines Patienten während einer Magnetresonanzuntersuchung mittels einer Magnetresonanzvorrichtung, wobei die Magnetresonanzvorrichtung eine Hochfrequenzantenne und eine Bewegungserfassungseinheit mit einer Radareinheit aufweist, mit den folgenden Schritten:
- einem Generieren zumindest eines Radarsignals mittels einer Radareinheit,
- einem Aussenden des zumindest einen generierten Radarsignals mittels der Hochfrequenzantenne,
- einem Erfassen zumindest eines reflektierten Radarsignals mittels der Hochfrequenzantenne und
- einem Auswerten des zumindest einen reflektierten Radarsignals zur Erfassung einer Bewegung des Patienten.

Hierdurch kann besonders einfach das Radarsignal zur Erfassung der Bewegung des Patienten an die Hochfrequenzantenne übertragen und von dort ausgesendet werden. Zudem kann die Bewegungserfassungseinheit besonders kompakt und Platz sparend innerhalb der Magnetresonanzvorrichtung, insbesondere innerhalb der Hochfrequenzantenneneinheit integriert werden. Das Aussenden und/oder das Empfangen der Radarsignale mittels der Hochfrequenzantenne kann zudem zeitgleich zu der Aussendung von Hochfrequenzsignalen erfolgen. Aufgrund der großen Frequenzunterschiede zwischen dem Hochfrequenzsignal, das eine Frequenz von 123,2 MHz aufweist, und den Radarsignalen, die eine Frequenz im Gigaherzbereich aufweisen, wird eine Störung und/oder unerwünschte Beeinflussung zwischen dem Hochfrequenzsignalen und den Radarsignalen verhindert.

Des Weiteren wird vorgeschlagen, dass das zumindest eine generierte Radarsignal mittels zumindest eines Einkopplungselements in zumindest eine Hochfrequenzleitung eingekoppelt wird, wobei mittels der zumindest einen Hochfrequenzleitung Hochfrequenzsignale an die Hochfrequenzantenne übertragen werden. Zudem wird das zumindest eine erfasste Radarsignal mittels zumindest eines Einkopplungselements aus der Hochfrequenzleitung ausgekoppelt. Hierdurch kann aufgrund der zumindest teilweisen Integration der Bewegungserfassungseinheit in die Hochfrequenzeinheit eine kompakte und Kosten günstige Bewegungserfassung erfolgen.

Besonders vorteilhaft werden unterschiedliche Radarsignale erfasst, die sich hinsichtlich einer Radarfrequenz und/oder hinsichtlich einer Hochfrequenzeinkoppelstelle in die Hochfrequenzantenne unterscheiden, wobei die unterschiedlichen Radarsignale zur Bestimmung einer Bewegung des Patienten miteinander verknüpft werden.

Die Vorteile des erfindungsgemäßen Verfahrens zur Erfassung einer Bewegung eines Patienten während einer Magnetresonanzuntersuchung entsprechen im Wesentlichen den Vorteilen der erfindungsgemäßen Magnetresonanzvorrichtung, welche vorab im Detail ausgeführt sind. Hierbei erwähnte Merkmale, Vorteile oder alternative Ausführungsformen können ebenso auch auf die anderen beanspruchten Gegenstände übertragen werden und umgekehrt.

Weitere Vorteile, Merkmale und Einzelheiten der Erfindung ergeben sich aus den im Folgenden beschriebenen Ausführungsbeispielen sowie anhand der Zeichnungen.

Es zeigen:
- Fig. 1: eine erfindungsgemäße Magnetresonanzvorrichtung in einer schematischen Darstellung,
- Fig. 2: eine Detailansicht einer Hochfrequenzeinheit und einer Bewegungserfassungseinheit der Magnetresonanzvorrichtung,
- Fig. 3: eine alternative Ausgestaltung der Bewegungserfassungseinheit in einer Detailansicht und
- Fig. 4: ein erfindungsgemäßes Verfahren zu einer Erfassung einer Bewegung eines Pateinten während einer Magnetresonanzuntersuchung.

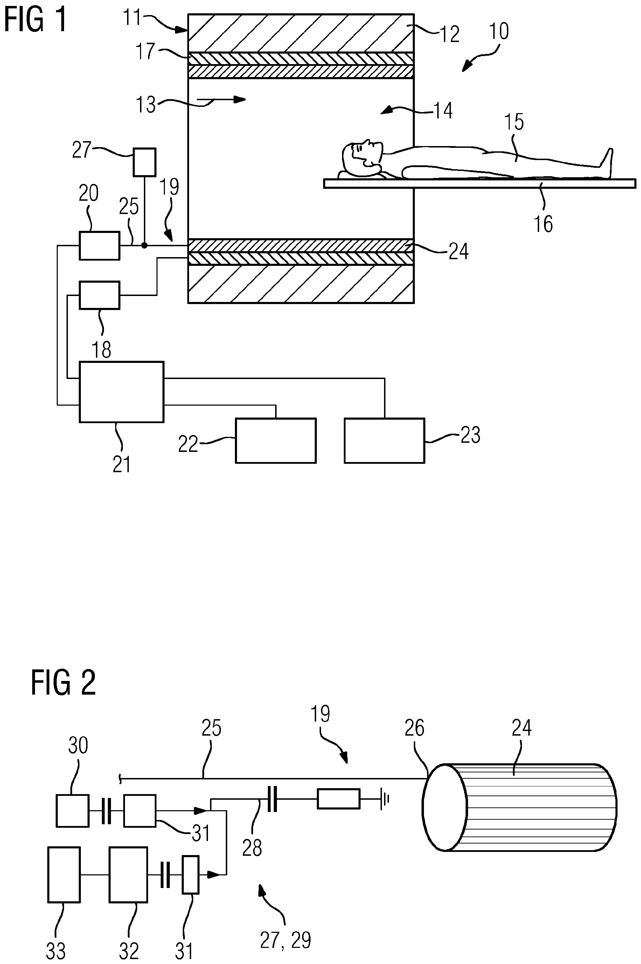

In Fig. 1 ist eine Magnetresonanzvorrichtung 10 schematisch dargestellt. Die Magnetresonanzvorrichtung 10 umfasst eine Magneteinheit 11 mit einem supraleitenden Hauptmagneten 12 zu einem Erzeugen eines starken und insbesondere konstanten Hauptmagnetfelds 13. Zudem weist die Magnetresonanzvorrichtung 10 einen Patientenaufnahmebereich 14 auf zu einer Aufnahme eines Patienten 15. Der Patientenaufnahmebereich 14 im vorliegenden Ausführungsbeispiel ist zylinderförmig ausgebildet und in einer Umfangsrichtung von der Magneteinheit 11 zylinderförmig umgeben. Grundsätzlich ist jedoch eine davon abweichende Ausbildung des Patientenaufnahmebereichs 14 jederzeit denkbar. Der Patient 15 kann mittels einer Patientenlagerungsvorrichtung 16 der Magnetresonanzvorrichtung 10 in den Patientenaufnahmebereich 14 geschoben werden.

Die Magneteinheit 11 weist weiterhin eine Gradientenspuleneinheit 17 zu einer Erzeugung von Magnetfeldgradienten auf, die für eine Ortskodierung während einer Bildgebung verwendet werden. Die Gradientenspuleneinheit 17 wird mittels einer Gradientensteuereinheit 18 der Magnetresonanzvorrichtung 10 gesteuert. Die Magneteinheit 11 umfasst weiterhin eine Hochfrequenzeinheit 19, die eine Hochfrequenzantenne 24 umfasst, und eine Hochfrequenzantennensteuereinheit 20 zu einer Anregung einer Polarisation, die sich in dem von dem Hauptmagneten 12 erzeugten Hauptmagnetfeld 13 einstellt. Die Hochfrequenzeinheit 19 wird von der Hochfrequenzantennensteuereinheit 20 gesteuert und strahlt hochfrequente Magnetresonanzsequenzen in einen Untersuchungsraum, der im Wesentlichen von einem Patientenaufnahmebereich 14 der Magnetresonanzvorrichtung 10 gebildet ist, ein. Innerhalb der Hochfrequenzsteuereinheit 20 werden auch die Hochfrequenzsignale generiert und mittels einer Hochfrequenzleitung 25 der Hochfrequenzeinheit 19 zur Hochfrequenzantenne 24 geleitet.

Zu einer Steuerung des Hauptmagneten 12, der Gradientensteuereinheit 18 und zur Steuerung der Hochfrequenzantennensteuereinheit 20 weist die Magnetresonanzvorrichtung 10 eine von einer Recheneinheit gebildete Steuereinheit 21 auf. Die Steuereinheit 21 steuert zentral die Magnetresonanzvorrichtung 10, wie beispielsweise das Durchführen einer vorbestimmten bildgebenden Gradientenechosequenz. Zudem umfasst die Steuereinheit 21 eine nicht näher dargestellte Auswerteeinheit zu einer Auswertung von Bilddaten. Steuerinformationen wie beispielsweise Bildgebungsparameter, sowie rekonstruierte Magnetresonanzbilder können auf einer Anzeigeeinheit 22, beispielsweise auf zumindest einem Monitor, der Magnetresonanzvorrichtung 10 für einen Bediener angezeigt werden. Zudem weist die Magnetresonanzvorrichtung 10 eine Eingabeeinheit 23 auf, mittels der Informationen und/oder Parameter während eines Messvorgangs von einem Bediener eingegeben werden können.

In Fig. 2 ist die Hochfrequenzeinheit näher dargestellt. Die Hochfrequenzeinheit weist eine Hochfrequenzantenne 24, zumindest eine Hochfrequenzleitung 25 und zumindest eine Hochfrequenzeinkoppelstelle 26 auf. Die Hochfrequenzeinheit 19 kann zudem auch mehr als eine Hochfrequenzleitung 25 und auch mehr als eine Hochfrequenzeinkoppelstelle 26 aufweisen, wobei vorzugsweise eine Anzahl der Hochfrequenzleitungen 25 einer Anzahl an Hochfrequenzeinkoppelstellen 26 entspricht. Die Hochfrequenzleitungen 25 umfassen im vorliegenden Ausführungsbeispiel Koaxialkabel. Grundsätzlich ist auch eine davon abweichende Ausbildung der Hochfrequenzleitungen 25 denkbar.

Mittels der Hochfrequenzleitung 25 werden Hochfrequenzsignale an die Hochfrequenzantenne 24 übertragen und in die Hochfrequenzantenne 24 an der Hochfrequenzeinkoppelstelle 26 eingespeist.

Die Magnetresonanzvorrichtung 10 weist des Weiteren eine Bewegungserfassungseinheit 27 auf zu einer Erfassung Bewegung eines innerhalb des Patientenaufnahmebereichs 14 positionierbaren Patienten 15. Die zu erfassende Bewegung des Patienten 15 ist vorzugsweise von einer Herzbewegung und/oder einer Atembewegung des Patienten 15 gebildet.

Die Hochfrequenzleitung 25 der Hochfrequenzeinheit 19 weist ein Einkopplungselement 28 auf, wobei mittels des Einkopplungselements 28 ein von der Bewegungserfassungseinheit 27 geniertes Signal, insbesondere ein Bewegungserfassungssignal, in die Hochfrequenzleitung 25 eingespeist wird und über die Hochfrequenzantenne 24 in den Patientenaufnahmebereich 14 abgestrahlt wird.

In Fig. 2 ist ein erstes Ausführungsbeispiel der Bewegungserfassungseinheit 27 dargestellt. Die Bewegungserfassungseinheit 27 umfasst eine von einer Dopplerradareinheit gebildete Radareinheit 29. Die Radareinheit 29 weist eine Radarsignalgenerierungseinheit 30 auf, die beispielsweise einen spannungsgesteuerten Oszillator umfasst. Das von der Radarsignalgenerierungseinheit 30 generierte Radarsignal wird mittels des Einkopplungselements 28, das von einem Richtkoppler gebildet ist, in die Hochfrequenzleitung 25 eingekoppelt. Das in die Hochfrequenzleitung 25 eingekoppelte Radarsignal wird in an der Hochfrequenzeinkoppelstelle 26 in die Hochfrequenzantenne 24 eingespeist und von der der Hochfrequenzantenne 24 in den Patientenaufnahmebereich 14 abgestrahlt.

Zwei sich gegenüberliegende Ports des Richtkopplers sind für das Hochfrequenzsignal ausgelegt. Ein weiterer Port des Richtkopplers ist für das Radarsignal zur Einkopplung in die Hochfrequenzleitung 25 ausgelegt. Ein vierter Port des Richtkopplers ist für einen Abschlusswiderstand vorgesehen.

Zudem weist die Bewegungserfassungseinheit 27 zwei Anpassungseinheiten 31 auf. Mittels der Anpassungseinheiten 31 kann der Richtkoppler, insbesondere bei einer Einkopplung und/oder einer Übertragung eines zu übertragenden Radarsignals, vorteilhaft an eine Frequenz des zu übertragenden Radarsignals angepasst werden. Die Anpassungseinheiten 31 können kapazitive Anpassungselemente und/oder induktive Anpassungselemente und/oder weitere, dem Fachmann als sinnvoll erscheinende Anpassungselemente aufweisen.

Das generierte Radarsignal wird von der Radarsignalgenerierungseinheit 30 über eine erste der zwei Anpassungseinheiten 31 an den Richtkoppler geleitet. Die erfassten Radarsignale werden von dem Richtkoppler über die zweite der zwei Anpassungseinheiten 31 an eine Signalerfassungseinheit 32 der Radareinheit 29 geleitet.

Das in den Patientenaufnahmebereich 14 eingestrahlte Radarsignal wird von dem Patienten 15, insbesondere von Organen des Patienten 15, reflektiert. Die reflektierten Radarsignale werden wiederum von der Hochfrequenzantenne 24 erfasst und über die Hochfrequenzeinkoppelstelle 16, die Hochfrequenzleitung 25 und dem Einkopplungselement 28, insbesondere dem Richtkoppler, an die Radareinheit 29 geleitet. In der Bewegungserfassungseinheit 27 werden die erfassten Radarsignale ausgewertet, wobei hierzu die Bewegungserfassungseinheit 27 eine Auswerteeinheit 33 aufweist, die der Signalerfassungseinheit 32 nachgeschaltet ist. Eine Bewegung von Organen, beispielsweise eine Herzbewegung und/oder eine Atembewegung, des Patienten 15 wird hierbei mittels des Dopplereffekts bestimmt. Hierzu werden auch die von der Radarsignalgenerierungseinheit 30 generierten Radarsignale an die Signalerfassungseinheit 32 übertragen. Alternativ oder zusätzlich kann die Auswertung der erfassten Radarsignale auch innerhalb der Steuereinheit 21 erfolgen und/oder die Auswerteeinheit der Bewegungserfassungseinheit 27 innerhalb der Steuereinheit 21 integriert sein.

Das generierte Radarsignal zur Erfassung der Herzbewegung und/oder der Atembewegung des Patienten 15 weist eine Frequenz von mindestens 3 GHz auf. Besonders vorteilhaft weist das Radarsignal eine Frequenz von mindestens 4 GHz und besonders bevorzugt von mindestens 5 GHz auf. Des Weiteren weist das Radarsignal eine maximale Frequenz von 30 GHz, vorteilhaft von 25 GHz und besonders bevorzugt von 15 GHz auf. Das Hochfrequenzsignal, das ebenfalls über die Hochfrequenzantenne 25 in den Patientenaufnahmebereich eingestrahlt wird, weist dagegen eine Frequenz von 123,2 MHz auf. Aufgrund der großen Frequenzunterschiede zwischen dem Hochfrequenzsignal und dem Radarsignal erfolgt ein zeitgleiches Aussenden der Hochfrequenzsignale und der Radarsignale im Wesentlichen störungsfrei.

Das Radarsignal zur Erfassung der Herzbewegung und/oder der Atembewegung des Patienten 15 ist zudem besonders schmalbandig ausgebildet. Vorzugsweise weist das schmalbandige Radarsignal eine Breite von nur einigen Hz. Eine Breite des schmalbandigen Radarsignals ist bevorzugt kleiner 100 Hz und besonders vorteilhaft kleiner 50 Hz.

Im vorliegenden Ausführungsbeispiel weist die Hochfrequenzeinheit 19 eine einzige Hochfrequenzeinkoppelstelle 26 und eine einzige Hochfrequenzleitung 25 mit einem einzigen Einkopplungselement 28 auf. Mittels der einzigen Hochfrequenzeinkoppelstelle 26 und der einzigen Hochfrequenzleitung 25 mit dem einzigen Einkopplungselement 28 werden zwei oder mehr unterschiedliche Radarsignale der Radareinheit 29 in die Hochfrequenzantenne 24 eingespeist. Die unterschiedlichen Radarsignale unterscheiden sich hinsichtlich einer Radarfrequenz. Hierzu werden mittels der Radarsignalgenerierungseinheit 30 Radarsignale mit unterschiedlichen Radarfrequenzen generiert.

Die in den Patientenaufnahmebereich 14 eingestrahlten unterschiedlichen Radarsignale werden innerhalb des Patientenaufnahmebereichs 14 reflektiert und/oder gestreut, insbesondere an dem Patienten 15 gestreut und/oder reflektiert, und mittels der Hochfrequenzantenne 24 erfasst und von der Auswerteeinheit 33 ausgewertet. Für eine Auswertung der Radarsignale werden die erfassten, unterschiedlichen Radarsignale miteinander verknüpft. Hierbei werden aus den Daten der erfassten, unterschiedlichen Radarsignale beispielsweise ein Quadratmittelwert und/oder weitere, dem Fachmann als sinnvoll erscheinende Werte berechnet. Zudem kann hierbei ein großes Verhältnis von Nutzsignal zu Störsignal bereitgestellt werden, da die Störsignale näherungsweise als statistisch unabhängig betrachtet werden können. Mittels der unterschiedlichen Radarfrequenzen kann die Bewegung des Patienten 15, insbesondere eine Herzbewegung und/oder eine Atembewegung, redundant aufgrund der reflektierten Radarsignale erfasst werden. Hierdurch können innerhalb der Übertragungsstrecke parasitäre Effekte und/oder Störeffekte, die beispielsweise für bestimmte Radarfrequenzen zu einer schlechten Transmission führen können, während der Erfassung und/oder der Auswertung der erfassten Radarsignale eliminiert werden.

Zur Auswertung der erfassten Radarsignale weist die Auswerteeinheit 33 eine nicht näher dargestellte Prozessoreinheit und eine entsprechende Auswertesoftware und/oder Computerprogramme auf. Die Auswertesoftware und/oder die Computerprogramme sind in einer nicht näher dargestellten Speichereinheit der Auswerteeinheit 33 gespeichert.

Alternativ zu einer separaten Auswerteeinheit 33 der Bewegungserfassungseinheit 27 kann die Auswertung der erfassten Radarsignale auch mittels der Auswerteeinheit der Steuereinheit 21 der Magnetresonanzvorrichtung 10 erfolgen und/oder die Auswerteinheit 33 der Bewegungserfassungseinheit 27 innerhalb der Steuereinheit 21 der Magnetresonanzvorrichtung 10 integriert sein.

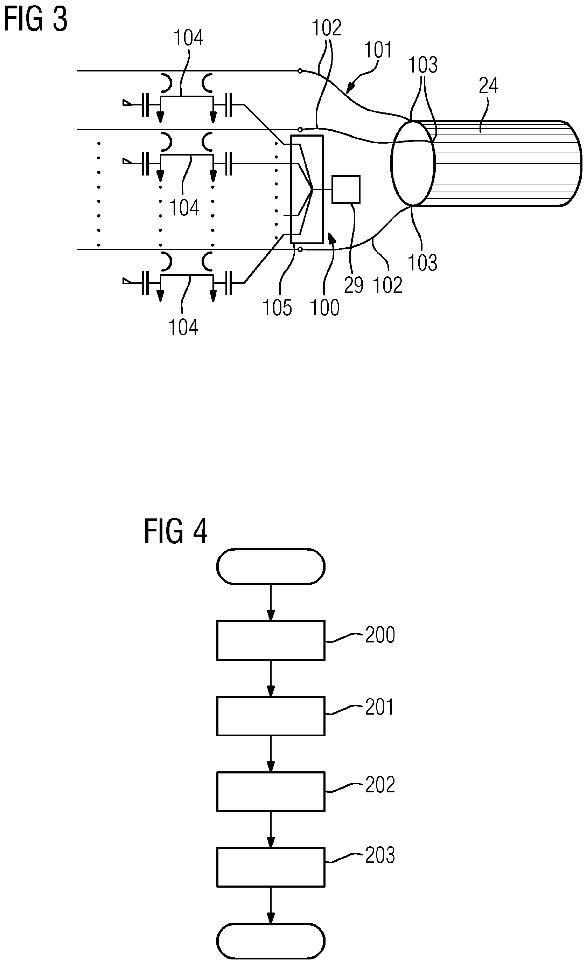

In Fig. 3 ist ein alternatives Ausführungsbeispiel der Bewegungserfassungseinheit 100 dargestellt. Im Wesentlichen gleich bleibende Bauteile, Merkmale und Funktionen sind grundsätzlich mit den gleichen Bezugszeichen beziffert. Die nachfolgende Beschreibung beschränkt sich im Wesentlichen auf die Unterschiede zu dem Ausführungsbeispiel in den Fig. 1 und 2, wobei bezüglich gleich bleibender Bauteile, Merkmale und Funktionen auf die Beschreibung des Ausführungsbeispiels in den Fig. 1 und 2 verwiesen wird.

Eine Hochfrequenzeinheit 101 der Magnetresonanzvorrichtung 10 weist eine Hochfrequenzantenne 24, zwei oder mehr Hochfrequenzleitungen 102 und zwei oder mehr Hochfrequenzeinkoppelstellen 103. Jede der zwei oder mehr Hochfrequenzleitungen 102 weist jeweils ein Einkopplungselement 104 auf. Im vorliegenden Ausführungsbeispiel umfassen die einzelnen Einkopplungselemente 104 jeweils einen Richtkoppler. Eine Anzahl der Hochfrequenzleitungen 102 entspricht hierbei einer Anzahl der Hochfrequenzeinkoppelstellen 103 in die Hochfrequenzantenne 24.

Die Bewegungserfassungseinheit 100 in Fig. 3 weist eine Radareinheit 29 auf, die analog zu der Beschreibung zu Fig. 2 ausgebildet ist. Zudem umfasst die Bewegungserfassungseinheit 100 eine Schalteinheit 105, die im vorliegenden Ausführungsbeispiel eine Mulitplexereinheit umfasst. Mittels der Multiplexereinheit erfolgt eine Selektionsschaltung zur Auswahl einer der Hochfrequenzleitungen 102 zur Einspeisung des Radarsignals. Eine Schaltfrequenz der Multiplexereinheit ist hierbei um mindestens einen Faktor 10 bis einen Faktor 100 größer als eine Atemfrequenz und/oder eine Herzfrequenz des Patienten 15. Derart erfolgt mittels jeweils eines Radarsignals, das an einer Hochfrequenzeinkoppelstelle 103 in die Hochfrequenzantenne 24 eingekoppelt wird, eine vorllständige Abtastung der Herzbewegung und/oder der Atembewegung des Patienten 15.

Mittels der unterschiedlichen Hochfrequenzeinkoppelstellen 103 werden die einzelnen Radarsignale mit einer zu dem Patienten 15 unterschiedlichen Richtung in den Patientenaufnahmebereich 14 eingestrahlt. Zudem ist es auch möglich, dass sich die einzelnen Radarsignale, die an den unterschiedlichen Hochfrequenzeinkoppelstellen 103 in die Hochfrequenzantenne 24 eingekoppelt werden, hinsichtlich einer Radarfrequenz unterscheiden. Hierzu werden von einer Radarsignalgenerierungseinheit 30 Radarsignale zur Einspeisung an den einzelnen Hochfrequenzeinkoppelstellen 103 erzeugt, wobei sich die Radarsignale an den einzelnen Hochfrequenzeinkoppelstellen 103 hinsichtlich der Radarfrequenz unterscheiden.

Eine Erfassung der von dem Patienten 15, insbesondere einem Herzbereich und/oder einem Lungenbereich des Patienten 15, reflektierten Radarsignalen und einer Weiterleitung der erfassten Radarsignale zu der Radareinheit 29 erfolgt mittels der Hochfrequenzeinheit 101 analog zu der Beschreibung zu Fig. 2. Die erfassten Radarsignale werden anschließend von der Auswerteeinheit 33 ausgewertet. Hierbei können die an den unterschiedlichen Hochfrequenzeinkoppelstellen 103 erfassten Radarsignale, die sich zudem hinsichtlich einer ausgesendeten Radarfrequenz unterscheiden können, miteinander verknüpft werden. Die Verknüpfung der einzelnen erfassten Radarsignale miteinander erfolgt analog zu der Beschreibung zu Fig. 2.

In Fig. 4 ist ein Verfahren zur Erfassung einer Bewegung eines Patienten 15 während einer Magnetresonanzuntersuchung mittels einer aus den Fig. 1 bis 3 bekannten Magnetresonanzvorrichtung 10 schematisch dargestellt. Die Magnetresonanzvorrichtung 10 weist hierzu eine Hochfrequenzantenne 24 und eine Bewegungserfassungseinheit 27, 100 mit einer Radareinheit 29 nach einem der Ausführungsbeispiele der Fig. 2 und 3 auf.

Nach einem Beginn des Verfahrens erfolgt in einem ersten Verfahrensschritt 200 ein Generieren eines Radarsignals mittels der Radarsignalgenerierungseinheit 30 der Radareinheit 29. Die Radarsignalgenerierungseinheit 30 kann hierbei unterschiedliche Radarsignale erzeugen, die sich hinsichtlich einer Radarfrequenz unterscheiden. Beispielsweise können hierbei von der Radarsignalgenerierungseinheit 30 eine vorgegebene Anzahl von Radarsignalen mit einer gleichmäßigen Verteilung der Radarfrequenzen zwischen beispielsweise 5 GHz und 15 GHz erzeugt werden. Vorzugsweise werden hierbei die unterschiedlichen Radarsignale nacheinander erzeugt, wobei nach einem jeweiligen Durchlauf die Radarsignalgenerierungseinheit 30 wieder von vorne beginnt. Alternativ hierzu kann die Radarsignalgenerierungseinheit 30 stets ein gleiches Radarsignal erzeugen, das fortlaufend in den Patientenaufnahmebereich 14 eingestrahlt wird.

In einem weiteren Verfahrensschritt 201 werden die generierten Radarsignale mittels der Hochfrequenzantenne 24 ausgesendet. Hierbei erfolgt vorher ein Einkoppeln der generierten Radarsignale in die Hochfrequenzeinheit 19, 101, insbesondere in die Hochfrequenzleitung 25, 102 mittels eines Einkopplungselements 28, 104. Je nach Ausgestaltung der Hochfrequenzeinheit 19, 101 stehen hierfür ein oder mehrere Einkopplungselemente 28, 104 zur Verfügung (siehe hierzu die Beschreibung zu Fig. 2 und 3). Mittels der Hochfrequenzantenne 24 werden die Radarsignale in den Patientenaufnahmebereich 14 eingestrahlt.

Nach dem Einstrahlen der Radarsignale in den Patientenaufnahmebereich 14 werden die eingestrahlten Radarsignale an dem Patienten 15 und/oder weiteren Objekten innerhalb des Patientenaufnahmebereichs 14 reflektiert und/oder gestreut. In dem Verfahrensschritt 202 werden anschließend die reflektierten Radarsignale mittels der Hochfrequenzantenne 24 erfasst. Die erfassten Radarsignale werden hierbei über die Hochfrequenzleitung 25, 102 zur Radareinheit 29 geleitet, wobei mittels des Einkopplungselements 28, 104 die erfassten Radarsignale aus der Hochfrequenzleitung 25, 102 ausgekoppelt werden. Innerhalb der Radareinheit 29 werden anschließend die erfassten Radarsignale zur Auswerteeinheit 33 geleitet.

In einem daran anschließenden Verfahrensschritt 203 werden die erfassten Radarsignale von der Auswerteeinheit 33 ausgewertet. Hierbei können zunächst die für eine Atembewegung und/oder eine Herzbewegung charakteristischen Signale von den restlichen, die Atembewegung und/oder die Herzbewegung überlagernden Signalen separiert werden. Zudem werden hierbei die erfassten Radarsignale mit den unterschiedlichen Radarfrequenzen und/oder die Radarsignale, die an unterschiedlichen Hochfrequenzeinkoppelstellen 103 in die Hochfrequenzantenne 24 eingespeist werden, von der Auswerteeinheit 33 verknüpft. Beispielsweise kann ein Quadratmittelwert berechnet werden und damit eine Verbesserung einer Erfassung einer Herzbewegung und/oder einer Atembewegung des Patienten 15 erzielt werden. Des Weiteren kann ein großes Verhältnis von Nutzsignal zu Störsignal bereitgestellt werden, da die Störsignale näherungsweise als statistisch unabhängig betrachtet werden können.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Magnetresonanzvorrichtung mit einer Hochfrequenzeinheit (19, 101), die eine Hochfrequenzantenne (24), zumindest eine Hochfrequenzleitung (25, 102) und zumindest eine Hochfrequenzeinkoppelstelle (26, 103) aufweist, wobei mittels der zumindest einen Hochfrequenzleitung (25, 102) Hochfrequenzsignale an die Hochfrequenzantenne (24) übertragen werden und an der zumindest einen Höchfrequenzeinkoppelstelle (26, 103) in die Hochfrequenzantenne (24) eingespeist werden,
einem Patientenaufnahmebereich (14), der zumindest teilweise von der Hochfrequenzantenne (24) umgeben ist, und einer Bewegungserfassungseinheit (27, 100) zu einer Erfassung einer Bewegung eines innerhalb des Patientenaufnahmebereichs (14) positionierbaren Patienten (15), wobei die Bewegungserfassungseinheit (27, 100) eine Radareinheit (29) aufweist und eine Bewegung des Patienten (15) mittels zumindest eines Radarsignals der Radareinheit (29) erfasst wird, wobei die Hochfrequenzsignale über die Hochfrequenzantenne (24) als hochfrequente Magnetresonanzsequenzen in den Patientenaufnahmebereich (14) eingestrahlt werden,
**dadurch gekennzeichnet, dass** die zumindest eine Hochfrequenzleitung (25, 102) zumindest ein Einkopplungselement (28, 104) aufweist, mittels dessen zumindest ein Bewegungserfassungssignal der Bewegungserfassungseinheit (27, 100) in die Hochfrequenzleitung (245, 102) einkoppelt,
und das zumindest eine Radarsignal zur Erfassung der Bewegung des Patienten (15) mittels der Hochfrequenzantenne (24) abgestrahlt wird.

2. Magnetresonanzvorrichtung nach einem der vorangehenden Ansprüche,
**dadurch gekennzeichnet, dass** zumindest ein reflektiertes Radarsignal mittels der Hochfrequenzantenne (24) erfasst wird, wobei das reflektierte Radarsignal an einem, in den Patientenaufnahmebereichs (14) einbringbarem Untersuchungsobjekt reflektiert wird.

3. Magnetresonanzvorrichtung nach einem der vorangehenden Ansprüche,
**dadurch gekennzeichnet, dass** das Radarsignal eine Frequenz von mindestens 3 GHz umfasst.

4. Magnetresonanzvorrichtung nach einem der vorangehenden Ansprüche,
**dadurch gekennzeichnet, dass** das Radarsignal eine Frequenz von maximal 30 GHz umfasst.

5. Magnetresonanzvorrichtung nach einem der vorangehenden Ansprüche,
**dadurch gekennzeichnet, dass** die Hochfrequenzeinheit (101) zwei oder mehr Hochfrequenzleitungen (102) und zwei oder mehr Hochfrequenzeinkoppelstellen (103) aufweist, wobei mittels der zwei oder mehr Hochfrequenzleitungen (102) jeweils ein Hochfrequenzsignal zu einer der zwei oder mehr Hochfrequenzeinkoppelstellen (103) übertragen wird und jede der zwei oder mehr Hochfrequenzleitungen (102) ein Einkopplungselement (104) zur Einspeisung eines Radarsignals in die Hochfrequenzantenne (24) aufweist.

6. Magnetresonanzvorrichtung nach Anspruch 5,
**dadurch gekennzeichnet, dass** die Bewegungserfassungseinheit (100) eine Schalteinheit (105) aufweist und die Einspeisung der zwei oder mehr Radarsignale in die Hochfrequenzantenne (24) mittels der Schalteinheit (105) erfolgt.

7. Magnetresonanzvorrichtung nach einem der vorangehenden Ansprüche,
**dadurch gekennzeichnet, dass** mittels einer einzigen Hochfrequenzeinkoppelstelle (26) und einer einzigen Hochfrequenzleitung (25) mit einem einzigen Einkopplungselement (28) zwei oder mehr Radarsignale in die Hochfrequenzantenne (24) eingespeist werden, wobei die zwei oder mehr Radarsignale hinsichtlich einer Radarfrequenz unterschiedlich ausgebildet sind.

8. Magnetresonanzvorrichtung nach einem der Ansprüche 5 bis 7,
**dadurch gekennzeichnet, dass** die Bewegungserfassungseinheit (27, 100) eine Auswerteeinheit (33) aufweist, die die unterschiedlichen, erfassten Radarsignale miteinander verknüpft werden.

9. Magnetresonanzvorrichtung nach einem der vorhergehenden Ansprüche,
**dadurch gekennzeichnet, dass** das Einkopplungselement (28, 104) einen Richtkoppler umfasst.

10. Magnetresonanzvorrichtung nach Anspruch 9,
**dadurch gekennzeichnet, dass** die Bewegungserfassungseinheit (27, 100) eine Anpassungseinheit (31) aufweist.

11. Magnetresonanzvorrichtung nach einem der vorhergehenden Ansprüche,
**dadurch gekennzeichnet, dass** die Bewegung des Patienten (15) eine Herzbewegung und/oder eine Atembewegung des Patienten (15) umfasst.

12. Verfahren zu einer Erfassung einer Bewegung, insbesondere einer Herzbewegung und/oder einer Atembewegung, eines Patienten (15) während einer Magnetresonanzuntersuchung mittels einer Magnetresonanzvorrichtung nach Anspruch 1 (10), wobei die Magnetresonanzvorrichtung (10) eine Hochfrequenzantenne (24) und eine Bewegungserfassungseinheit (27, 100) mit einer Radareinheit (29) aufweist, mit den folgenden Schritten:
- einem Generieren zumindest eines Radarsignals mittels einer Radareinheit (29),
- einem Aussenden des zumindest einen generierten Radarsignals mittels der Hochfrequenzantenne (24),
- einem Erfassen eines zumindest eines reflektierten Radarsignals mittels der Hochfrequenzantenne (24) und
- einem Auswerten des zumindest einen reflektierten Radarsignals zur Erfassung einer Bewegung des Patienten (15),
- einem Aussenden hochfrequenter Magnetresonanzsequenzen mittels der Hochfrequenzantenne (24).

13. Verfahren nach Anspruch 12,
**dadurch gekennzeichnet, dass** das zumindest eine generierte Radarsignal mittels zumindest eines Einkopplungselements (28, 104) in zumindest eine Hochfrequenzleitung (25, 102) eingekoppelt wird, wobei mittels der zumindest einen Hochfrequenzleitung (25, 102) Hochfrequenzsignale an die Hochfrequenzantenne (24) übertragen werden.

14. Verfahren nach einem der Ansprüche 12 bis 13,
**dadurch gekennzeichnet, dass** das zumindest eine erfasste Radarsignal mittels zumindest eines Einkopplungselements (28, 104) aus einer Hochfrequenzleitung (25, 102) ausgekoppelt wird, wobei mittels der zumindest einen Hochfrequenzleitung (25, 102) Hochfrequenzsignale an die Hochfrequenzantenne (24) übertragen werden.

15. Verfahren nach einem der Ansprüche 12 bis 14,
**dadurch gekennzeichnet, dass** unterschiedliche Radarsignale erfasst werden, die sich hinsichtlich einer Radarfrequenz und/oder hinsichtlich einer Hochfrequenzeinkoppelstelle (26, 103) in die Hochfrequenzantenne (24) unterscheiden, wobei die unterschiedlichen Radarsignale zur Bestimmung einer Bewegung des Patienten (15) miteinander verknüpft werden.

## Claims

1. Magnetic resonance device having a radiofrequency unit (19, 101) which has a radiofrequency antenna (24), at least one radiofrequency line (25, 102) and at least one radiofrequency injection point (26, 103), wherein radiofrequency signals are transferred to the radiofrequency antenna (24) by means of the at least one radiofrequency line (25, 102) and injected into the radiofrequency antenna (24) at the at least one radiofrequency injection point (26, 103), a patient receiving zone (14) which is at least partially enclosed by the radiofrequency antenna (24), and a motion detection unit (27, 100) for detecting a movement of a patient (15) that can be positioned within the patient receiving zone (14), wherein the motion detection unit (27, 100) has a radar unit (29) and a movement of the patient (15) is detected by means of at least one radar signal of the radar unit (29), wherein the radiofrequency signals are radiated via the radio frequency antenna (24) into the patient receiving zone (14) as radiofrequency magnetic resonance sequences,
**characterised in that** the at least one radiofrequency line (25, 102) has at least one injection element (28, 104) by means of which at least one motion detection signal of the motion detection unit (27, 100) is coupled into the radiofrequency line (245, 102),
and the at least one radar signal for detecting the movement of the patient (15) is emitted by means of the radiofrequency antenna (24).

2. Magnetic resonance device according to one of the preceding claims,
**characterised in that** at least one reflected radar signal is acquired by means of the radiofrequency antenna (24), the reflected radar signal being reflected off an examination subject that can be introduced into the patient receiving zone (14) .

3. Magnetic resonance device according to one of the preceding claims,
**characterised in that** the radar signal has a frequency of at least 3 GHz.

4. Magnetic resonance device according to one of the preceding claims,
**characterised in that** the radar signal has a maximum frequency of 30 GHz.

5. Magnetic resonance device according to one of the preceding claims,
**characterised in that** the radiofrequency unit (101) has two or more radiofrequency lines (102) and two or more radiofrequency injection points (103), wherein a radiofrequency signal is transferred in each case to one of the two or more radiofrequency injection points (103) by means of the two or more radiofrequency lines (102) and each of the two or more radiofrequency lines (102) has an injection element (104) for injecting a radar signal into the radiofrequency antenna (24).

6. Magnetic resonance device according to claim 5,
**characterised in that** the motion detection unit (100) has a switching unit (105) and the two or more radar signals are injected into the radiofrequency antenna (24) by means of the switching unit (105).

7. Magnetic resonance device according to one of the preceding claims,
**characterised in that** two or more radar signals are injected into the radiofrequency antenna (24) by means of a single radiofrequency injection point (26) and a single radiofrequency line (25) having a single injection element (28), wherein the two or more radar signals are embodied differently in terms of a radar frequency.

8. Magnetic resonance device according to one of claims 5 to 7,
**characterised in that** the motion detection unit (27, 100) has an evaluation unit (33) which combines the different acquired radar signals with one another.

9. Magnetic resonance device according to one of the preceding claims,
**characterised in that** the injection element (28, 104) comprises a directional coupler.

10. Magnetic resonance device according to claim 9,
**characterised in that** the motion detection unit (27, 100) has an adapter unit (31).

11. Magnetic resonance device according to one of the preceding claims,
**characterised in that** the movement of the patient (15) comprises a cardiac motion and/or a respiratory motion of the patient (15).

12. Method for detecting a movement, in particular a cardiac motion and/or a respiratory motion, of a patient (15) during a magnetic resonance examination by means of a magnetic resonance device (10) according to claim 1, wherein the magnetic resonance device (10) has a radiofrequency antenna (24) and a motion detection unit (27, 100) having a radar unit (29), said method comprising the following steps:
- generating at least one radar signal by means of a radar unit (29),
- transmitting the at least one generated radar signal by means of the radiofrequency antenna (24),
- acquiring at least one reflected radar signal by means of the radiofrequency antenna (24), and
- evaluating the at least one reflected radar signal for the purpose of detecting a movement of the patient (15),
- transmitting radiofrequency magnetic resonance sequences by means of the radiofrequency antenna (24).

13. Method according to claim 12,
**characterised in that** the at least one generated radar signal is coupled into at least one radiofrequency line (25, 102) by means of at least one injection element (28, 104), wherein radiofrequency signals are transferred to the radiofrequency antenna (24) by means of the at least one radiofrequency line (25, 102).

14. Method according to one of claims 12 to 13,
**characterised in that** the at least one acquired radar signal is coupled out of a radiofrequency line (25, 102) by means of at least one injection element (28, 104), wherein radiofrequency signals are transferred to the radiofrequency antenna (24) by means of the at least one radiofrequency line (25, 102).

15. Method according to one of claims 12 to 14,
**characterised in that** different radar signals are acquired which differ from one another in terms of a radar frequency and/or in terms of a radiofrequency injection point (26, 103) into the radiofrequency antenna (24), wherein the different radar signals are combined with one another in order to determine a movement of the patient (15).

## Revendications

1. Dispositif de résonance magnétique, comprenant une unité (19, 101) de haute fréquence, qui a une antenne (24) de haute fréquence, au moins une ligne (25, 102) de haute fréquence et au moins un point (26, 103) de couplage de haute fréquence, dans lequel, au moyen de la au moins une ligne (25, 102) de haute fréquence, des signaux de haute fréquence sont transmis à l'antenne (24) de haute fréquence et sont injectés dans l'antenne (24) de haute fréquence en le au moins un point 26, 103) de couplage de haute fréquence,
une partie (14) de réception de patient, qui est entourée, au moins en partie, de l'antenne (14) de haute fréquence et une unité (27, 100) de détection de mouvement pour détecter un mouvement d'un patient (15) mis en position dans la partie (24) de réception d'un patient, dans lequel l'unité (27, 100) de détection de mouvement a une unité (29) de radar et un mouvement du patient (15) est détecté au moyen d'au moins un signal radar de l'unité (29, de radar,
dans lequel les signaux de haute fréquence arrivent par l'intermédiaire de l'antenne (24) de haute fréquence, sous la forme de séquences de résonance magnétique de haute fréquence dans la partie (14) de réception d'un patient **caractérisé en ce que** la au moins une ligne (25, 102) de haute fréquence a au moins un élément (28, 104) d'injection, au moyen duquel au moins un signal de détection de mouvement de l'unité (27, 100) de détection de mouvement est injecté dans la ligne (245, 102) de haute fréquence,
et le au moins un signal radar est, pour la détection du mouvement du patient (15), émis au moyen de l'antenne (24) de haute fréquence.

2. Dispositif de résonance magnétique suivant l'une des revendications précédentes,
**caractérisé en ce qu'**au moins un signal radar réfléchi est détecté au moyen de l'antenne (24) de haute fréquence, le signal radar réfléchi étant réfléchi sur un objet à étudier, pouvant être mis dans la partie (14) de réception d'un patient.

3. Dispositif de résonance magnétique suivant l'une des revendications précédentes,
**caractérisé en ce que** le signal radar comprend une fréquence d'au moins 3 GHz.

4. Dispositif de résonance magnétique suivant l'une des revendications précédentes,
**caractérisé en ce que** le signal radar comprend une fréquence d'au maximum 30 GHz.

5. Dispositif de résonance magnétique suivant l'une des revendications précédentes,
**caractérisé en ce que** l'unité (101) de haute fréquence a deux ou plusieurs lignes (102) de haute fréquence et deux ou plusieurs points (103) de couplage de haute fréquence, dans lequel, au moyen de deux ou plusieurs lignes (102) de haute fréquence, respectivement, un signal de haute fréquence est transmis à l'un des deux ou plusieurs points (103) de couplage de haute fréquence et chacune des deux ou plusieurs lignes (102) de haute fréquence a un point (104) de couplage pour injecter un signal radar dans l'antenne (24) de haute fréquence.

6. Dispositif de résonance magnétique suivant la revendication 5,
**caractérisé en ce que** l'unité (100) de détection de mouvement a une unité (105) de commutation et l'injection de deux ou plusieurs signaux radar dans l'antenne (24) de haute fréquence s'effectue au moyen de l'unité (105) de commutation.

7. Dispositif de résonance magnétique suivant l'une des revendications précédentes,
**caractérisé en ce qu'**au moyen d'un point (26) de couplage de haute fréquence unique et d'une ligne (25) de haute fréquence unique, ayant un élément (28) de couplage unique, deux ou plusieurs signaux radar sont injectés dans l'antenne (24) de haute fréquence, les deux ou plusieurs signaux radar étant constitués différemment du point de leur fréquence radar.

8. Dispositif de résonance magnétique suivant l'une des revendications 5 à 7,
**caractérisé en ce que** l'unité (27, 100) de détection de mouvement a une unité (33) d'exploitation, qui combine entre eux les signaux radar différents détectés.

9. Dispositif de résonance magnétique suivant l'une des revendications précédentes,
**caractérisé en ce que** l'élément (28, 104) d'injection comprend un coupleur directionnel.

10. Dispositif de résonance magnétique suivant la revendication 9,
**caractérisé en ce que** l'unité (27, 100) de détection de mouvement a une unité (31) d'adaptation.

11. Dispositif de résonance magnétique suivant l'une des revendications précédentes,
**caractérisé en ce que** le mouvement du patient (15) comprend un mouvement du coeur et/ou un mouvement de respiration du patient (15).

12. Procédé de détection d'un mouvement, notamment d'un mouvement du coeur et/ou d'un mouvement de respiration d'un patient (15) pendant un examen par résonance magnétique au moyen d'un dispositif (10) de résonance magnétique suivant la revendication 1, dans lequel le dispositif (10) de résonance magnétique a une antenne (24) de haute fréquence et une unité (27, 100) de détection du mouvement, ayant une unité (29) radar, comprenant les stades suivants :
- une production d'au moins un signal radar au moyen d'une unité (29) radar,
- une émission du au moins un signal radar produit au moyen de l'antenne (24) de haute fréquence,
- une détection d'au moins un signal radar réfléchi au moyen de l'antenne (24) de haute fréquence et
- une exploitation du au moins un signal radar réfléchi pour détecter un mouvement du patient (15),
- une émission de séquences de résonance magnétique de haute fréquence au moyen de l'antenne (24) de haute fréquence.

13. Procédé suivant la revendication 12,
**caractérisé en ce que** l'on injecte le au moins un signal radar, produit au moyen d'au moins un élément (28, 104) de couplage, dans au moins une ligne (25, 102) de haute fréquence, des signaux de haute fréquence étant transmis à l'antenne (24) de haute fréquence au moyen de la au moins une ligne (25, 102) de haute fréquence.

14. Procédé suivant l'une des revendications 12 à 13,
**caractérisé en ce que** le au moins un signal radar détecté est découplé d'une ligne (25, 102) de haute fréquence au moyen d'un élément (28, 104) de couplage, dans lequel des signaux de haute fréquence sont transmis à l'antenne (24) de haute fréquence au moyen de la au moins une ligne (25, 102) de haute fréquence.

15. Procédé suivant l'une des revendications 12 à 14,
**caractérisé en ce que** l'on détecte des signaux radar différents, qui diffèrent du point de vue d'une fréquence radar et/ou du point de vue d'un point (26, 103) de couplage de haute fréquence dans l'antenne (24) de haute fréquence, les signaux radar différents étant combinés entre eux pour déterminer un mouvement du patient (15).
